# EUROPEAN PATENT SPECIFICATION

(11) **EP 1 198 237 B1**
(45) Date of publication and mention of the grant of the patent: **09.06.2004**
(21) Application number: 00958780.9
(22) Date of filing: 04.09.2000
(51) Int. Cl.: A61K 35/78, A61P 17/10, A61P 17/00

(54) **HERBAL COMPOSITIONS FOR ITCHY OR INFECTED SKIN**
KRÄUTERZUSAMMENSETZUNGEN FÜR JUCKREIZ ODER INFIZIERTE HAUTTEILE
COMPOSITIONS A BASE D'HERBES POUR PEAUX INFECTEES OU PRURIGINEUSES

(30) Priority: 03.09.1999 GB 9920886
(43) Date of publication of application: 24.04.2002
(73) Proprietor: Shah, Eladevi, London NW11 9SY (GB)
(72) Inventor: Shah, Eladevi, London NW11 9SY (GB)
(86) International application number: PCT/GB2000/003383
(87) International publication number: WO 2001/017539

(56) References cited:
- GB-A- 2 274 058
- US-A- 5 693 327

## Description

This invention relates to the manufacture and use of herbal compositions for the treatment of various skin disorders itchy or infected skin such as impetigo, acne (on face, forehead scalp and on the back of the body) and fungal infection of skin and nails.

### Acne

Acne is a disease of the pilosebaceous unit. It predominantly affects the face, back and chest.
Perioral area of the face is more vulnerable. Still acne is not a simple face skin disorder, few patients with severe acne on the back or chest who have non on the face.

Acne is a mixture of, inflammation- infection process and favoured by the presence of time oriented hormonal imbalance.

Most commonly found anaerobes and/or aerobe- bacteria in the acne are *Staphylococcus albus*, *Staphylococcus epidermidis, Pitirosporum ovale Propionaebacterium acnes.*

One gentleman suffering from acne on his face and also on his back for years. He was reluctant for antibiotics because he knew that antibiotics gave more trouble. He came to me for natural treatment for keloids he was suffering for long time. I gave him oral compound and herbal cream to apply one month supply, on 25th day of treatment he came to me for cream supply. At that time I counted spots on his back out of 33 spots 25 spots were healed. I realised that this is Propionae bacterium has disappeared. Now I would like to present a laboratory proof here.

### Types of the lesions

### Comedones are of two types. Open (black heads), closed (whiteheads)

Open blackheads contain greyish greasy content of the pilosebaceous duct which is affected by acne process. Some blackheads develop into inflamed lesions.

Closed comedones (whiteheads) are caused by a distension of the pilosebaceous duct and these are more likely than open comedones (blackheads) to develop into inflamed lesions.
About a quarter of closed comedones spontaneously resolve within 3-4 days while the majority progress to become inflamed. Some whiteheads persist in a latent state for months.

### Facial scarring

### Acne and immune response

As previously stated acne is a mixture of, inflammation-infection process and favoured by the presence of time oriented hormonal imbalance.

Pustules resolve faster than papules. In pustules natural healing process is faster because of polymorphonucleocyte activity and in papules the natural healing process is slower because here it depends on lymphocyte activity. Here this lymphocyte activity remains helpful in further growth of the comedones. Macules are the end result of inflamed lesions, most persist for 7 days and they can linger for several weeks.
Present invention is useful for prevention of further scarring.

### Facial scarring

It is well recognised that facial scarring and scars on the back are associated with long standing changing guards-games played by different sorts of bacteria fungi and immune response of the body in the form of production and presentation of lymphocyte and polymorphonucleocytes.

### Fungal nail infection and immune response

The skin in some people react to external and internal changes very fast such as drinking alcohol, washing with alkaline soap, increased sweating in summer and more humid skin environment. All these skin sensitising factors could bring circulatory changes on the skin and inside the nail beds.
It is well recognised that alkalinity on the skin initiates or promotes fungal infections. In long journey of antibiotics, resistance development and hide and seek games played by organisms the present invention remains most blissful.

The skin and blood purifier herbal composition for oral and topical treatments for Acne and fungal infections of skin and the nails

### Oral composition

Centratherum anthelminticum, *Curcuma longa, Melia azardirachta, Picrorrhyza curroa, Cassia tora, Comiphora mukul, Withenia somnifera, Tinospora cordifolia, Asparagus racemosus, Triphala.*

### Topical treatment

Centratherum *anthelminticum, Melia azadirachta, Cassia tora*.

### Mouth wash

### Centratherum anthelminticum, Melia azadirachta, Cassia tora

### Face wash and body wash

### Centratherum anthelminticum, Melia azadirachta, Cassia tora

### Nasal drops

### Centratherum anthelminticum, Melia azadirachta, Cassia tora

### SUMMARY OF THE INVENTION

According to a first aspect of the present invention, there is provided a composition for use in the treatment of skin disorders itchy or infected skin such as impetigo, acne (on face, forehead scalp and on the back of the body) and fungal infection of skin and nails, which composition comprises extracts derived from the seeds of the plant *Centratherum anthelminthicum.*

According to second aspect of the present invention, there is provided a composition for use in the treatment of skin disorders such as acne and /or fungal infection and/ or fungal infections of the nails, which composition comprises extracts derived from the seeds of the plant *Centratherum anthelminthicum* fruit-oil and extract from leaves, bark, roots of the plant Melia azadirachta.

According to third aspect of the present invention, there is provided a composition for use in the treatment of skin disorders such as acne and /or fungal infection and/ or fungal infections of the nails, which composition comprises extracts derived from the seeds of the plant *Centratherum anthelminthicum* fruit-oil and extract from leaves, bark, roots of the plant *Melia azadirachta* and extracts derived from the seeds of the plant *Cassia tora*.

According to fourth aspect of the present invention, there is provided a composition for use in the treatment of skin disorders such as acne and /or fungal infection and/ or fungal infections of the nails, which composition comprises extracts derived from at least one member of the group consisting of the plants *Centratherum anthelminticum, Melia azardirachta, Cassia tora* which further comprises extracts derived from *Picrorrhyza curroa, Comiphora mukul, Withenia somnifera, Tinospora cordifolia, Asparagus racemosus, Triphala.*

According to fifth aspect of the present invention, there is provided a method of treating itchy skin or infected skin through the administration of extracts derived from at least one member of the group consisting of the plants *Centratherum anthelminticum*, *Melia azadirachta, Cassia tora.*

According to a sixth aspect of the present invention, there is provided a method of treating itchy and infected skin through the administration of extracts derived from at least one member of the group consisting of the plants *Centratherum anthelminticum, Melia azadirachta, Cassia tora* and further extracts derived from at least one member of the group consisting of the plants *Picrorrhyza curroa, Comiphora mukul, Withenia somnifera, Tinospora cordifolia, Asparagus racemosus, Triphala.*

According to a seventh aspect of the present invention, there is provided a method of treating infected skin through the administration of extracts derived from at least one member of the group consisting of the plants *Centratherum anthelminticum, Melia azadirachta, Cassia tora.*

According to an eighth aspect of the present invention, there is provided a method of treating acne and fungal infections of skin and the nails through the administration of extracts derived from at least one member of the group consisting of the plants *Centratherum anthelminticum, Melia azadirachta, Cassia tora* and further extracts derived from at least one member of the group consisting of the plants *Picrorrhyza curroa, Comiphora mukul, withenia somnifera, Tinospora cordifolia, Asparagus racemosus, Triphala.*

The composition of the present invention may be dispersed in a suitable carrier for topical application as a cream, gel, ointment or lotion, or may be in the form of a dry powder, or other form suitable for a portable decoction, for example by way of a tea bag. Alternatively, the composition may be formulated in a tablet form, or composition may be formulated and made spray dried extract.

Preferably, the composition spray dried extract is dispersed in a carrier suitable for topical application comprising ghee, which is clarified butter.

The compositions of the present invention help to break the cycles of pruritus-inflamation-pruritus, and of infection-inflamation-pruritus, thereby promoting faster healing of acute, sub-acute and chronic bacterial and fungal infection of the skin and fungal of nails. The compositions of the present invention are additionally useful in reducing the effects of stress and reinforcing immune response.

Extracts from *Centratherum anthelminticum, Melia azadirachta* disperse in a sesame oil and cream was formulated with ghee and antimicrobial preservative efficacy test was performed using *Propionibacterium* acnes NCTC 737 was introduced in a cream Inoculum count cfu/ml 2.1 x 10⁵ and significant results were seen logarithm reduction unto 5.32 was achieved at the 7 days inoculation test and 14 days inoculation tests.

Extract from *Cassia* tora dispersed well with yogurt and heated and then applied on impetigo worked well. the activity was dose dependent.

### Example 1

A preferred embodiment of the present invention is a cream made up as follows:

| | |
|---|---|
| Ghee | 3kg |
| Water boiled and cooled freezing temperature 3litres *F*reeze dried extract of *Centratherum anthelminticum 5* gms (2 kilogram raw material of of the seeds provided 100 grams of freeze dried powder) | |
| Medicated Sesame oil | 500 ml |

(500 ml sesame oil treated with *Centratherum anthelminticum* seeds 100gm, Rose petals 100gm, *Melia azadirachta* leaves 100 gm, *Cassia tora* seeds 50 gm and some water to make a paste}.

To make up the cream, the ghee is first hydrolysed in a clean stainless steel vessel with three litres of water (boiled and cooled freezing ) then add 500 ml of medicated oil.

### Example 2

A preferred embodiment of the present invention is a cream made up as follows:

| | |
|---|---|
| Ghee | 3kg |
| Water boiled and cooled freezing temperature 3litres Freeze dried extract of Centratherum anthelminticum 5 gms (2 kilogram raw material of of the seeds provided 100 grams of freeze dried powder) | |
| Medicated Sesame oil | 500 ml |

(500 ml sesame oil treated with *Centratherum anthelminticum* seeds 100gm, Rose petals 100gm, *Melia azadirachta* leaves 100 gm, and some water to make a paste).

To make up the cream, the ghee is first hydrolysed in a clean stainless steel vessel with three litres of water (boiled and cooled freezing )then add 500 ml of medicated oil.

## Claims

1. The use in the manufacture of a medicament for the treatment of impetigo, acne and fungal infections of the skin and nails of an extract from seeds of the plant *Centratherum anthelminticum.*

2. The use in the manufacture of claim 1, together with said extract, of fruit oil and an extract derived from a part of the plant *Melia azadirachta.*

3. The use according to claim 2, wherein the *Melia azadirachta* extract is derived from leaves, bark or roots of the plant *Melia azadirachta.*

4. The use according to any preceding claim, wherein, included in the medicament, is an extract derived from seeds of the plant *Cassia tora.*

5. The use according to any preceding claim, wherein, included in the medicament, is one or more further extracts, the or each further extract being derived from *Picrorrhiza kurroa, Comiphora mukul, Withenia somnifera, Tinospora cordifolia, Asparagus racemosus* and *Triphala.*

6. A pharmaceutical composition for use in the treatment of skin disorders and fungal infections of the skin and nails, which comprises herbal extracts derived from *Centratherum anthelminticum* and *Cassia tora*, the herbal extracts being dispersed in a suitable carrier for topical application or being in the form of a dry powder for the preparation of a potable decoction, a mouthwash or nasal drops.

7. A composition as claimed in claim 6, which additionally contains a herbal extract derived from *Melia azadirachta.*

8. A composition according to claim 6 to 7, which additionally comprises a herbal extract derived from one or more of *picrorrhyza curroa, Comiphora mukul*, *Withenia somnifera*, *Tinospora cordifolia, Asparagus racemosus* and *Triphala.*

9. A pharmaceutical composition for use in the treatment of skin disorders and fungal infections of skin and nails, which comprises the herbal extracts derived from *Centratherum anthelminticum, Melia azadirachta* and one or more of *Comiphora mukul*, *Withenia somnifera* and *Triphala,* the herbal extracts being dispersed in a suitable carrier for topical application or being in the form of a dry powder for the preparation of a potable decoction, a mouthwash or nasal drops.

10. A composition as claims in any claims 6 to 9 in which the powder is a spray dried or freeze dried powder or is for topical application and includes Ghee as carrier.

## Patentansprüche

1. Das Benutzen eines Extraktes von der Saat der Pflanze Centratherum Athelminticum zum Herstellen eines Medikamentes für die Behanlung von Impetigo, Akne und Pilz-Infizierung von Haut und Nägeln.

2. Der Gebrauch im Herstellen gemäss von Anspruch I. zusammen mit einem erwähnten Extrakt von Frucht-Öl und einem Extrakt von einem Teil der Pflanze Melia Azadirachta.

3. Der Gebrauch in Übereinstimmung mit Anspruch 2. wo ein Extrakt von Melia azadirachta dass von Blättern, Borke, oder von der Wurzel der Pflanze Melia azadirachta stammt.

4. Der Gebrauch in Übereinstimmung mit jedem vorhergehenden Anspruch, wo ein Extrakt von der Saat der Pflanze Cassia tora im Medikament eingeschlossen ist.

5. Der Gebrauch im Einstimmen mit jedem vorhergehenden Anspruch,wo ein oder mehrere Extrakte in das Medikament mit eingeschlossen sind, der Extrakt oder mehrere Extrakte von Picrorrhiza kurroa,Comiphora mukul, Withenia somnifera,Tinospora cordifalia,Asparagus racemosus und Triphala herstammen.

6. Ein pharmazeutisches Zusammensetzen zur Behandlung von Haut Erkrankgungen und Pilz Infizierungen der Haut und Nägeln, die aus Kräuter-Extrekten bestehen, die von Centra anthelminticum und Cassiatora herkommen und wo die Krauter Extrakte in einem geeigneten Träger zum aktuellen Gebrauch eingestreut werden oder als ein trockenes Puder zur Vorbereitung eines trinkbaren Dekoktes um M und-Waschen oder als Nasen-Tropfen bereit gestellt wird.

7. Ein Zusammenstellen gemäss des Anspruches 6 dass zusätzlich einen Kräuter Extrakt enthält den man vonMelia azadirachta erhält.

8. Ein Zusammenstellen gemäss der Ansprüche 6 zu 7 welches zusätzlich einen Kräuter Extrakt enthält den man von einem oder mehreren picrorrhyza curroa, comiphora mukul, Withenia somnifera, Tinospora cordifolia, Asparagus racemosus und Triphala, erhält.

9. Ein pharmazeutisches Zusammenstellen für den Gebrauch in der Behandlung von Haut Krankheiten und Pilz Infizierungen von Haut und Nägeln, dass Kräuter Extrakte enthält, die von Centratherum anthelminticum,Melia azadirachta, und eine oder mehr Comiphora mukul, Withenia somnifera und Triphala herstammen. die Kräuter Extrakte werden in einem geeigneten Träger für aktuelle Benutzung gestreut oder sie werden als ein trockenes Puder zur Vorbereitung eines trinkbare Dekoktes, zum Mundwaschen oder für Nasen-Tropfen benutzt.

10. Eine Zusammenstellung gemäss der Ansprüche 6 zu 9 wo das Puder Spritzgetrocknet oder gefrier getrocknet ist oder es ist für eine aktuelle Benutzung bestimmt und enthält Ghee als einen Träger.

## Revendications

1. Utilisation, dans la fabrication d'un médicament pour le traitement de l'impétigo, de l'acné et des infections fongiques de la peau et des ongles, d'un extrait de la graine de la plante *Centratherum anthelminticum.*

2. Utilisation, dans la fabrication en Revendication 1, ajouté à l'extrait en question, d'huile de fruit et d'un extrait dérivé d'une partie de la plante *Melia azadirachta.*

3. Utilisation suivant revendication 2 où l'extrait de *Melia azadirachta* est obtenu des feuilles, de l'écorce et de la racine de la plante Melia *azadirachta*.

4. Utilisation suivant toutes revendications précédentes, par laquelle, compris dans le médicament, il y a un extrait dérivé des graines de la plante *Cassia tora.*

5. Utilisation suivant toute précédente revendication, par laquelle, compris dans le médicament, il y a un ou plusieurs autres extraits, cet extrait ou tout autre étant dérivé de *Picrorrhiza kurroa, Comiphora mukul, Withenia somnifera, Tinospora cordifolia, Asparagus racemosus* et *Triphala.*

6. Une composition pharmaceutique à utiliser dans le traitement des affections de la peau et les infections fongiques de la peau et des ongles, qui comprend des extraits herbiers tirés de *Centratherum anthelminticum* et *Cassia tora,* les extraits herbiers étant dispersés dans un support adapté à l'application topique ou se présentant sous forme de poudre sèche pour la préparation de décoction liquide, rince bouche ou gouttes dans le nez.

7. Une composition comme il est indiqué en revendication 6, qui contient en outre un extrait herbier dérivé de *Melia azadirachta.*

8. Une composition suivant Revendication 6 à 7, qui contient en outre un extrait herbier dérivé de un ou plusieurs des produits suivants : *picrorrhyza curroa, Comiphora mukul, Withenia somnifera, Tinospora cordifolia, Asparagus racemosus* et *Triphala.*

9. Une composition pharmaceutique à utiliser dans le traitement des affections de la peau et les infections fongiques de la peau et des ongles, qui comprend les extraits herbiers dérivés de *Centratherum anthelminticum, Melia azadirachta* et un ou plusieurs des produits suivants *Comiphora mukul, Withenia somnifera* et *Triphala,* étant dispersés dans un support adapté à une application topique ou se présentant sous forme de poudre sèche pour la préparation de décoction liquide, rince bouche ou gouttes dans le nez.

10. Une composition comme il est indiqué en Revendications 6 à 9 dans laquelle la poudre est desséchée par nébulisation ou lyophilisée ou est destinée à une application topique et utilise le Ghee comme support.
